# DEMANDE DE BREVET EUROPEEN

(11) **EP 2 082 736 A1**
(43) Date de publication de la demande: **29.07.2009**
(21) Numéro de dépôt: 08150554.7
(22) Date de dépôt: 23.01.2008
(51) Int. Cl.: A61K 31/00, A61K 31/407, A61P 17/00

(54) **Composition pharmaceutique à usage topique**

(71) Demandeur: Saurat, Jean Hilaire, 1206 Genève (CH)
(72) Inventeur: Saurat, Jean Hilaire, 1206 Genève (CH)
(74) Mandataire: Bugnion Genève

(57) **Abrégé**

Utilisation, en tant que médicament dermatologique, de composés susceptibles d'interagir avec le récepteur AHR (Aryl Hydrocarbon Receptor) en tant qu'agents modulateurs de fonctions cutanées telles que la fonction sébacée, la défense anti-infectieuse, la cicatrisation de la peau, l'atrophie de la peau dite « dermatoporose », et la déprivation oestrogénique, sans induire d'autres effets toxiques de type TCDD.

Les composés interagissant avec le récepteur AHR sont choisis en ce qu'ils ont un métabolisme favorable à la dissociation de ces effets, en particulier grâce à la production in situ à partir d'un précurseur et/ou la métabolisation modulée in situ.

## Description

La présente invention concerne une composition pharmaceutique à usage topique, destinée au traitement et/ou à la prévention de dysfonctionnements de fonctions cutanées. La présente invention concerne plus spécifiquement une composition pharmaceutique à usage topique, destinée au traitement et/ou à la prévention de dysfonctionnements de la fonction sébacée, de la défense anti-infectieuse, de la cicatrisation, de l'atrophie dite « dermatoporose (Ref Dermatology 2007.215. 284-294) », et de la déprivation oestrogénique de la peau.

Il existe déjà des traitements des dysfonctionnements mentionnés ci-dessus, mais qui ne sont toutefois pas exempts de défauts. Ainsi, à titre d'exemples :
- On sait qu'un dérivé de la vitamine A, l'isotrétinoïne (ou acide rétinoïque 13-cis), est actif dans le traitement de maladies telles que l'hyperséborrhée et l'acné en induisant une atrophie des glandes sébacées. Mais cette substance doit être administrée par voie systémique et non par voie topique, car par voie topique elle n'atrophie pas la glande sébacée.
- Pour renforcer les capacités de défense anti-infectieuse de sujets porteurs d'une forte susceptibilité aux infections cutanées bactériennes et/où fongiques, on a déjà proposé l'utilisation d'antibiotique au long cours, mais cette méthode présente le défaut de sélectionner des souches résistantes et de ne pas augmenter les défenses intrinsèques du sujet.
- Pour faciliter la cicatrisation de plaies cutanées, il est connu de protéger les tissus en cours de reconstruction, notamment au moyen de pansements hydrocolloïdes, mais cette technique assure une protection mécanique superficielle sans stimuler spécifiquement les processus intrinsèques de reconstitution des tissus proprement dits, lorsqu'il existe un défaut de production de facteurs de croissance, notamment vasculaires.
- On a proposé de traiter la dermatoporose au moyen de rétinoïdes et de fragments d'acide hyaluronique, mais les premiers sont irritants et les seconds difficiles à préparer dans leurs taille et viscosité optimale.
- Dans le cas de la déprivation oestrogènique, il est connu d'administrer des oestrogènes par voie systémique, ou topique. Mais cet apport d'hormones endogènes présente l'inconvénient de modifier dans son ensemble l'équilibre hormonal du patient et, selon plusieurs publications, d'augmenter le risque d'apparition de certains cancers.

Il reste donc un besoin non satisfait de développer de nouveaux médicaments contre les dysfonctionnements mentionnés ci-dessus et en particulier des compositions pharmaceutiques à usage topique permettant d'éviter les inconvénients liés à une administration systémique.

Les composés interagissant avec le récepteur AhR (Aryl Hydrocarbon Receptor), dont le prototype est la 2,3,7,8 tetrachloro-dibenzo-*p*-dioxine (TCDD) sont pour la plupart des xéno-toxiques, induisant différents types de lésions tissulaires et autres manifestations d'intoxication, dépendantes de la dose et de l'espèce. Parmi ces manifestations de l'intoxication à la dioxine, la peau occupe une place majeure, en partie en raison de l'expression notoire du récepteur AhR par les cellules épitheliales et mesenchymales de la peau. C'est pourquoi l'utilisation thérapeutique et/ou préventive de tels composés en tant qu'agents actifs, modulateurs de fonctions cutanées semble à priori exclue.

Lors de l'intoxication humaine par TCDD, un syndrome complexe est observé au niveau de la peau. L'étude approfondie de ce syndrome chez l'homme et chez l'animal d'expérience a conduit le demandeur à analyser les mécanismes cellulaires et moléculaires de constitution de ces lésions toxiques. De façon surprenante, le demandeur a découvert certains effets favorables de l'exposition à ces composés toxiques, décrits ci-dessous, ainsi que les moyens de les obtenir, en l'absence des effets toxiques mentionnés plus haut, pour moduler des fonctions cutanées, en particulier la fonction sébacée, la défense anti-infectieuse, la cicatrisation, l'atrophie de la peau dite « dermatoporose (Ref Dermatology 2007.215. 284-294) », et la déprivation oestrogénique.

En conséquence, l'invention concerne des composés agonistes du récepteur AhR et/ou les protéines impliquées dans ses voies d'activation cellulaires, ces composés agissant dans le cadre de l'invention en tant qu'agents modulateurs de fonctions cutanées, en particulier de la fonction sébacée, de la défense anti-infectieuse, de la cicatrisation, de l'atrophie dite « dermatoporose », et de la déprivation oestrogénique de la peau.

L'invention a pour objet une composition pharmaceutique à usage topique, destinée à induire de façon transitoire l'activation de récepteurs AhR cytosoliques des tissus cutanés, ladite composition comprenant une substance active choisie parmi les ligands agonistes de AhR métabolisables par les enzymes de phase 1 et parmi les précurseurs in situ des dits ligands.

La présente invention est donc totalement distincte, et même antinomique, de certaines inventions de l'art antérieur qui ont proposé l'utilisation de ligands antagonistes_du récepteur AhR, en vue d'empêcher une expression génique du type de celles susceptibles d'être induites par une exposition à TCDD. Ces inventions, décrites notamment dans les documents W02004/041758, W02007/060256 et W02007/128725, étaient basées sur des extrapolations tirées d'expériences in vitro et sur le postulat que l'activation de la voie AhR serait par définition néfaste. Au contraire le demandeur a découvert que l'activation chez l'homme et l'animal d'expérience de la voie AhR peut avoir des effets favorables pour le traitement de maladies telles que les anomalies de la fonction sébacée, de la défense anti-infectieuse, de la cicatrisation, l'atrophie dite « dermatoporose », et la déprivation oestrogénique de la peau.

La toxicité de ligands xéno-toxiques de AhR, tels que la TCDD, apparaît essentiellement due au fait que ces substances ne sont pratiquement pas métabolisées par l'organisme humain, dans les tissus duquel elles présentent des demi-vies de l'ordre de 4-12 ans. De ce fait, les molécules de TCDD se lient de façon répétitive au complexes AhR cytosoliques, provoquant leur dissociation, leur translocation dans le noyau cellulaire, la dimérisation avec ARNT et une transcription génique devenant progressivement anarchique. Au contraire, grâce à l'emploi de ligands de AhR métabolisables, notamment par les enzymes de phase 1, dont la durée de demi-vie dans l'organisme s'évalue en jours, l'activation de AhR résulte en une activation bénéfique de certaines fonctions cutanées.

Les ligands mis en oeuvre dans le cadre de la présente invention ont de préférence des durées de demie vie dans l'organisme humain compris entre 1 et 60 jours, et, plus spécifiquement, et selon l'application, entre 3 et 30 jours.

Il convient de ne pas utiliser de ligand métabolisable de AhR dont un métabolite serait lui-même toxique et non catabolisé. Dans cette optique, la substance active peut être choisie parmi les ligands endogènes de AhR.

D'une façon générale la dissociation entre effet bénéfique selon l'invention et effets toxiques est obtenue en stimulant le récepteur AhR d'une façon modulée et transitoire. Cette dissociation peut être obtenue par la formation d'un ligand *in situ* à partir d'un précurseur administré par voie topique. Ladite substance active peut être un précurseur choisi parmi les proligands métaboliques de ligands de AhR. Ladite substance active peut également être un précurseur activable en ligand de AhR sous l'effet d'un agent physique, en particulier sous l'effet d'un rayonnement UV.

L'invention sera mieux comprise de l'homme du métier grâce à la description ci après de plusieurs applications spécifiques, en se référant aux exemples correspondants et aux figures accompagnantes, dans lesquelles
- la figure 1 montre la disparition des glandes sébacées en présence de TCDD chez l'homme ;
- la figure 2 montre des souris traitées respectivement par application de TCDD et d'un ligand selon l'invention ;
- la figure 3 montre la réduction des glandes sébacées par application topique de TCDD et de FICZ ;
- les figures 4a et 4b montrent la colonisation de la peau humaine respectivement par staphylocoque sp. et par p-acnes, en l'absence et en cas d'exposition à la TCDD ;
- la figure 5 montre la colonisation de la peau humaine par staphylocoque sp. dans le cas d'un traitement par un ligand selon l'invention ;
- les figures 6a et 6b montre les temps de cicatrisation de plaies superficielles en l'absence et en cas d'exposition à la TCDD ;
- la figure 7 montre des temps de cicatrisation de plaies traitées par TCDD et par un ligand selon l'invention ;
- la figure 8 montre l'expression des ARN messagers de facteurs de croissance de peau exposée a la TCDD ;
- la figure 9 montre l'expression d'ARN messagers de récepteurs oestrogéniques et de protéines associées en cas d'exposition à la TCDD ;
- la figure 10 montre la concentration d'oestradiol dans la peau humaine exposée à la TCDD.

En ce qui concerne les glandes sébacées, l'effet utile de la TCDD, au sens de la présente invention, est la diminution progressive de la taille des glandes. Cet effet peut être reproduit chez l'animal par application topique de TCDD. Cet effet est spécifique car il n'y a pas chez l'homme d'atrophie des autres structures annexielles telles que poils et glandes sudorales. Ainsi la présente invention utilise l'effet d'inhibition spécifique de la glande sébacée par un ligand de AhR pour la prévention et le traitement des maladies comportant une hypertrophie de ces glandes, telles que l'hyperséborrhée et l'acné, en induisant une atrophie des glandes sébacées. Cette invention offre pour cette application un spectre d'activité identique à celui de l'isotrétinoïne, mais par voie topique. Il est également possible d'envisager une application thérapeutique contre les tumeurs des glandes sébacées et leurs analogues, tels que les carcinomes à différentiation sébacée du syndrome de Muir-Torre.

Les ligands AhR utilisés pour reproduire cet effet, sont choisis sur la base d'une réduction de plus de 50% de la taille et du nombre des glandes sébacées, sans induire de signes toxiques : Les moyens pour obtenir l'effet sur les glandes sébacées sans induire d'autres effets toxiques de type TCDD, notamment la formation des stuctures hamartomateuses (Acquired Dioxin Induced Skin Hamartomas, ADISH), consistent en l'utilisation de composés interagissant transitoirement avec le récepteur AhR, de sorte que la cinétique soit favorable à cette dissociation temporelle entre effet favorable et effets toxiques.

En ce qui concerne la défense anti-infectieuse, l'effet utile de la TCDD constaté par le demandeur se manifeste par une résistance marquée aux infections cutanées bactériennes, fungiques, et sans doute virales. Cette résistance coïncide avec une stabilisation permanente de la flore de surface au bénéfice d'espèces saprophytes non pathogènes. Cette résistance se traduit par l'absence de surinfection par des germes pathogènes. Elle est parallèle et probablement liée en partie à une surexpression de défensines, dont la béta défensine. Elle est aussi parallèle et sans doute liée au moins en partie à une surexpression de filaggrine. La filaggrine est une protéine de différentiation épidermique dont le défaut d'expression est fortement lié à la dermatite atopique, une maladie caractérisée par une forte susceptibilité aux infections cutanées bactériennes, notamment staphylococciques.

Un objet de l'invention est donc une composition pharmaceutique administrable par voie topique destinée à traiter et prévenir les infections cutanées bactériennes et/ou fungiques notamment chez les sujets porteurs d'une forte susceptibilité telle que la dermatite atopique, ainsi que d'autres situations de portage chronique, cette composition comprenant au moins un composé interagissant en tant qu'agoniste avec le récepteur AhR. Les moyens pour obtenir l'effet désiré sur les infections cutanées sans induire d'autres effets toxiques de type TCDD consistent en l'utilisation de composés dont la cinétique d'interaction avec le récepteur AhR soit favorable à cette dissociation : l'invention propose en particulier la production *in situ* à partir d'un précurseur tel que le tryptophane ( TRP ) ou un dérivé de TRP, administré dans un véhicule pharmaceutique permettant la pénétration transcutanée, suivi d'une exposition à un rayonnement UV, l'interaction avec le récepteur AhR étant limitée par la métabolisation *in situ* par des enzymes de phase 1.

En ce qui concerne la déprivation oestrogénique, l'effet utile de la TCDD se manifeste par une activation des voies de production des oestrogènes ainsi que par une surexpression des récepteurs oestrogéniques. Les oestrogènes sont des hormones endogènes très importantes dans de nombreuses fonctions cutanées; la déprivation oestrogénique entraîne des troubles de chacune de ces fonctions.

Un objet de l'invention est donc une composition pharmaceutique administrable par voie topique destinée à traiter et prévenir les troubles des fonctions cutanées liés à une déprivation oestrogénique, cette composition comprenant au moins un composé interagissant en tant qu'agoniste avec le récepteur AhR. Les moyens pour obtenir l'effet désiré sur l'expression d'oestrogènes sans induire d'autres effets toxiques de type TCDD consistent en l'utilisation de composés dont la cinétique d'interaction avec le récepteur AhR soit favorable à cette dissociation : L'invention propose en particulier la production *in situ* à partir d'un précurseur tel que le tryptophane ( TRP ) ou un dérivé de TRP, administré dans un véhicule pharmaceutique permettant la pénétration transcutanée, suivi d'une exposition à un rayonnement UV, l'interaction avec le récepteur AhR étant limitée par la métabolisation *in situ* par des enzymes de phase 1.

En ce qui concerne la cicatrisation et l'atrophie de la peau dite « dermatoporose (Ref Dermatology 2007.215. 284-294) » l'effet utile de la TCDD constaté par le demandeur se manifeste par une impressionnante rapidité de cicatrisation des plaies. Ainsi, le demandeur a observé que des plaies épidermo-dermiques dont le temps de cicatrisation moyen est de 10 jours, étaient recouvertes en 3 jours. Cet effet est en rapport avec l'induction par la TCDD de plusieurs facteurs de croissance (Vascular Endothelial Growth Factor VEGF, Amphireguline). L'effet de la TCDD est encore caractérisé par une très importante accumulation de hyaluronate dans le derme, parallèle à l'hyper-expression des hyaluronate-synthases (voir figures).

Un objet de l'invention est donc une composition pharmaceutique administrable par voie topique destinée à traiter et prévenir les troubles des fonctions cutanées liés à des troubles et retards de la cicatrisation cutanée, cette composition comprenant au moins un composé interagissant en tant qu'agoniste avec le récepteur AhR. Les moyens pour obtenir l'effet désiré sur la cicatrisation cutanée sans induire d'autres effets toxiques de type TCDD consistent en l'utilisation de composés dont la cinétique d'interaction avec le récepteur AhR soit favorable à cette dissociation: l'invention propose en particulier la production *in situ* à partir d'un précurseur tel que le tryptophane ( TRP ) ou un dérivé de TRP, administré dans un véhicule pharmaceutique permettant la pénétration transcutanée, suivi d'une exposition à un rayonnement UV, l'interaction avec le récepteur AhR étant limitée par la métabolisation *in situ* par des enzymes de phase 1.

Un autre objet de l'invention est donc une composition pharmaceutique administrable par voie topique destinée à traiter et prévenir l'atrophie cutanée dite dermatoporose (Ref Dermatology 2007.215. 284-294), qui est caractérisée par une déplétion de hyaluronate conduisant à une perte de la viscoelasticité de la peau, cette composition comprenant au moins un composé interagissant en tant qu'agoniste avec le récepteur AhR, du type décrit ci-dessus.

Les composés qui se lient à AhR et sont susceptibles de produire les effets selon l'invention comprennent des
- Hydrocarbures aromatiques polycycliques
   a. 2- ou 6-Azulénol; rétinoate ou linoléate de 2- ou 6-azulényle
   b. 2-, 3-, 5-, 6- ou 8-Guaïazulénol; rétinoate ou linoléate de 2-, 3-, 5-, 6- ou 8-guaïazulényle
   c. rétinoïde guaïazulénylique : acide 3,7-diméthyl-9-[1-[3,8-diméthyl-5-isopropyl-azulényl]]-2,4,6,8-nonatétraènoïque
- Flavonoïdes
   d. Flavanols avec substituants alkyle, alkényle, alcoxyle ou carboxyle en positions 4 à 6 et 3' à 5'
   e. Flavanones avec substituants alkyle, alkényle, alcoxyle ou carboxyle en positions 5, 6 et 3' à 5'
   f. Flavones avec substituants alkyle, alkényle, alcoxyle ou carboxyle en positions 3, 5, 6 et 3' à 5'
   g. Flavonols avec substituants alkyle, alkényle, alcoxyle ou carboxyle en positions 5, 6 et 3' à 5'
   h. Isoflavones avec substituants alkyle, alkényle, alcoxyle ou carboxyle en positions 5, 6 et 3' à 5'
   i. Anthocyanidines avec substituants alkyle, alkényle, alcoxyle ou carboxyle en positions 4 à 6 et 3' à 5'
   j. Gallate d'épigallocatéchine; esterification en l'une ou l'autre des positions 5, 7, 3' à 5', 3" à 5" avec acides gras ou acide rétinoïque
- Stilbènes
   k. *cis*-Stilbènes avec substituants hydroxyle, alkyle, alkényle, alcoxyle ou carboxyle en positions 1 à 5 et 1' à 5'
   1. *trans*-Stilbènes avec substituants hydroxyle, alkyle, alkényle, alcoxyle ou carboxyle en positions 1 à 5 et 1' à 5'
- Dérivés du tryptophane
   m. Indole-3-carbinol; dérivés alkyle, alkényle, alcoxyle ou carboxyle sur le groupe hydroxyle
   n. Tryptamine
   o. Kynurénine et ester du groupe carboxylique avec rétinol
   p. Acide indole acétique et ester du groupe carboxylique avec rétinol
   q. Acide indole pyruvique et ester du groupe carboxylique avec rétinol
   r. 6-Formylindolo[3,2-b]carbazole et dérivés alkyle ou alkényle du groupe formyle
   s. 3,3'-diindolylméthane
   t. Trythantrine
   u. Malassézine
   v. Indirubine
   w. Indigo
- Autres ligands
   x. ITE [2-(1'h-indole-3'-carbonyl)-thiazole-4-carboxylate de méthyle] (endogène, voir US 20020183524)
   y. YH439 {(Isopropyl-2-(1,3-dithioethane-2-ylidene)-2-[N-(4-methylthiazol-2-yl) carbamoyl] acétate}
   z. a-Naphthoflavone
   aa. b-Naphthoflavone

Parmi les Ligands de AhR particulièrement préférés, on peut citer
bb. 6-Formylindolo[3,2-b]carbazole et dérivés alkyle ou alkényle du groupe formyle
cc. Malassézine
dd. Indirubine
ee. Isoflavones avec substituants alkyle, alkényle, alcoxyle ou carboxyle en positions 5, 6 et 3' à 5'
ff. Gallate d'épigallocatéchine; esterification en l'une ou l'autre des positions 5, 7, 3' à 5', 3" à 5" avec acides gras ou acide rétinoïque
gg. 2-, 3-, 5-, 6- ou 8-Guaïazulénol; rétinoate ou linoléate de 2-, 3-, 5-, 6- ou 8-guaïazulényle.

Le document W02007/060256 décrit une méthode générale de test in vitro, permettant de déterminer si une substance donnée présente ou non des propriétés de ligand agoniste ou antagoniste de AhR. Elle permet donc de présélectionner les substances candidates à une utilisation dans le cadre de la présente invention et son enseignement est incorporé par référence à la présente demande.

Les méthodes pour délivrer le ligand du recepteur AhR dans les condition optimales pour l'effet désiré comportent
a. L'application directe du ligand actif dans un véhicule galénique approprié tel que lotion, crème, pommade, mousse.
b. L'application topique, dans un véhicule galénique approprié, d'un proligand, qui sera métabolisé en ligand par les enzymes épidermiques telles que esterases, etc...
c. L'application d'un proligand à la surface de la peau à traiter, qui sera activé en ligand par l'effet d'un agent physique tel que les UV ; par exemple Tryptophane et UV.

### Exemple 1 : Glandes sébacées

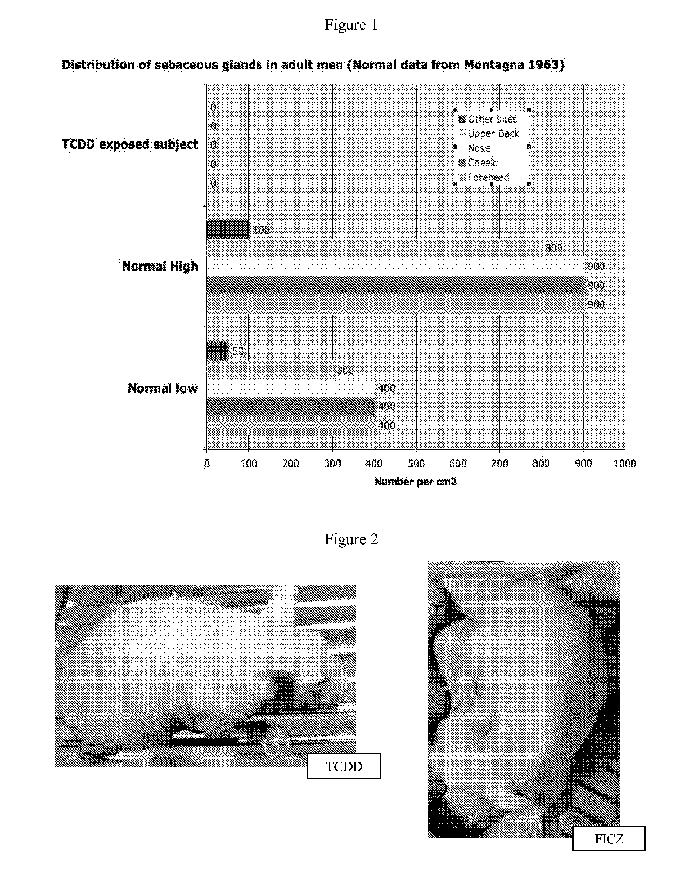

Les glandes sébacées en grand nombre dans la peau humaine normale disparaissent lors de l'exposition à des doses toxiques de TCDD. La figure 1 montre le nombre de glandes sébacées par centimètre carré sur différentes zones du corps humain, qui se distribue de 50 à 900 selon les zones et les sujets. Ainsi sur le visage d'un adulte, le nombre est compris entre 400 et 900 / cm2.

La figure 1 montre que lors de l'exposition à TCDD les glandes sébacées disparaissent dans toutes les zones étudiées. Pour chaque site, 3 à 10 biopsies de la peau ont été analysées après fixation au formol et coloration par hématéine-éosine. Au moins 10 sections par bloc ont été analysées. Sur un total de 62 biopsies, totalisant 620 sections, aucune glande sébacée n'a pu être observée, alors que les glandes sudorales ainsi que les poils étaient en nombre et structure conservée. Ces données ont été obtenues sur des spécimens de peau humaine contenant de 930 à 2900 ppt de TCDD mesurée par chromatographie gazeuse/spectrographie de masse.

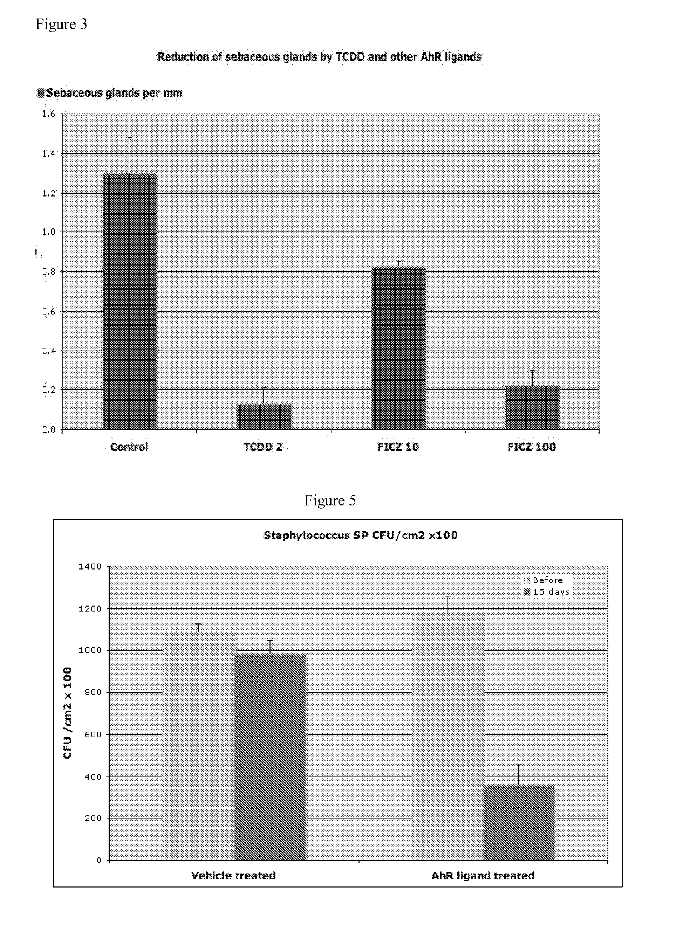

L'effet sur les glandes sébacées peut être reproduit chez l'animal par l'application topique de TCDD. La figure 3 montre la réduction de 90% du nombre des glandes sébacées chez des souris après 50 jours d'application de TCDD 2 mcg/ml. On observe également chez ces souris traitées par TCDD une importante irritation de la peau (figure 2). Pour reproduire l'effet utile du TCDD, on peut utiliser un ligand de AhR tel que le 6-Formylindolo[3,2-b]carbazole (FICZ) par voie topique. L'application de FICZ à une concentration optimale de 100 à 800 mcg/ml permet d'obtenir une diminution significative des glandes sébacées (figure 3). Contrairement au TCDD, le FICZ n'entraine pas d'effets toxiques (figure 2)

### Exemple 2 :Défense anti-infectieuse

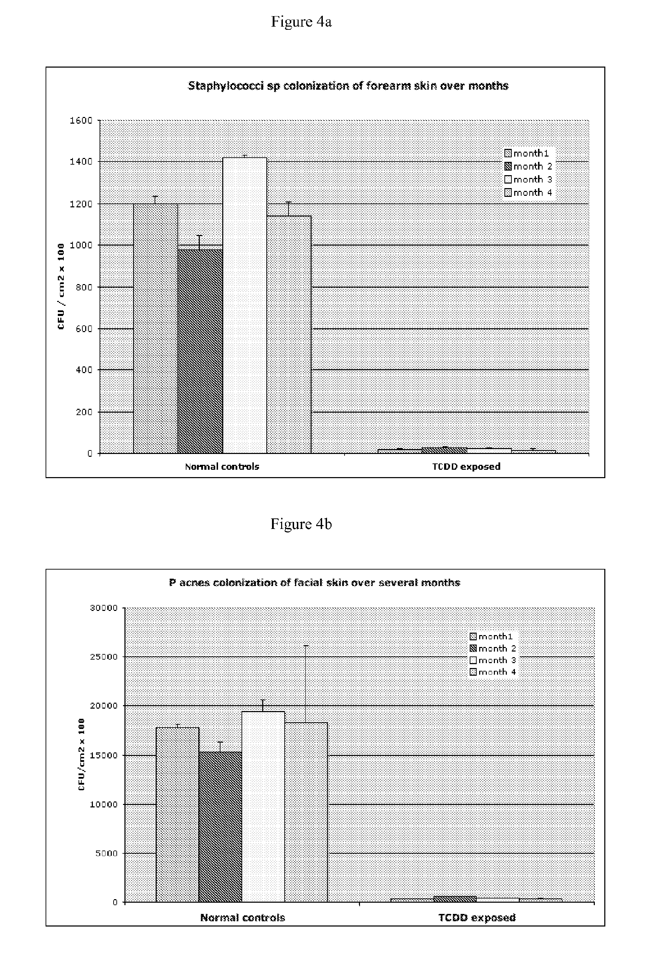

Comme le montre la figure 4a, la colonisation de la peau humaine par le **staphyloccoque sp** (nombre de CFU, colony forming units, par cm2) est très fortement diminuée en cas d'exposition à TCDD. Cette figure montre les valeurs obtenues sur 4 prélèvements à 1 mois d'intervalle chez un sujet humain exprimant plus de 900 ppt de TCDD dans la peau, mesurée par chromatographie gazeuse/spectrographie de masse. Le même effet est observé sur la colonisation par p-acnes, et illustré par la figure 4b. La figure 5 montre que la colonisation de la peau humaine par le staphyloccoque sp ( CFU, colony forming units, par cm2) est très diminuée par l'application topique d'un ligand du récepteur AhR, le 6-Formylindolo[3,2-b]carbazole (FICZ 300 mcg/ml).

### Exemple 3 : Cicatrisation

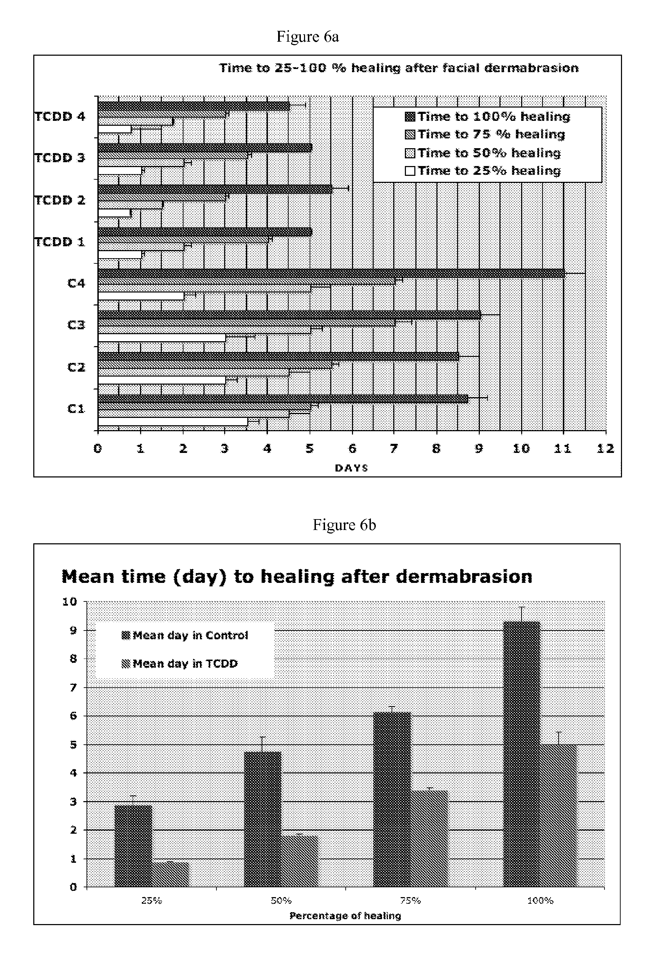

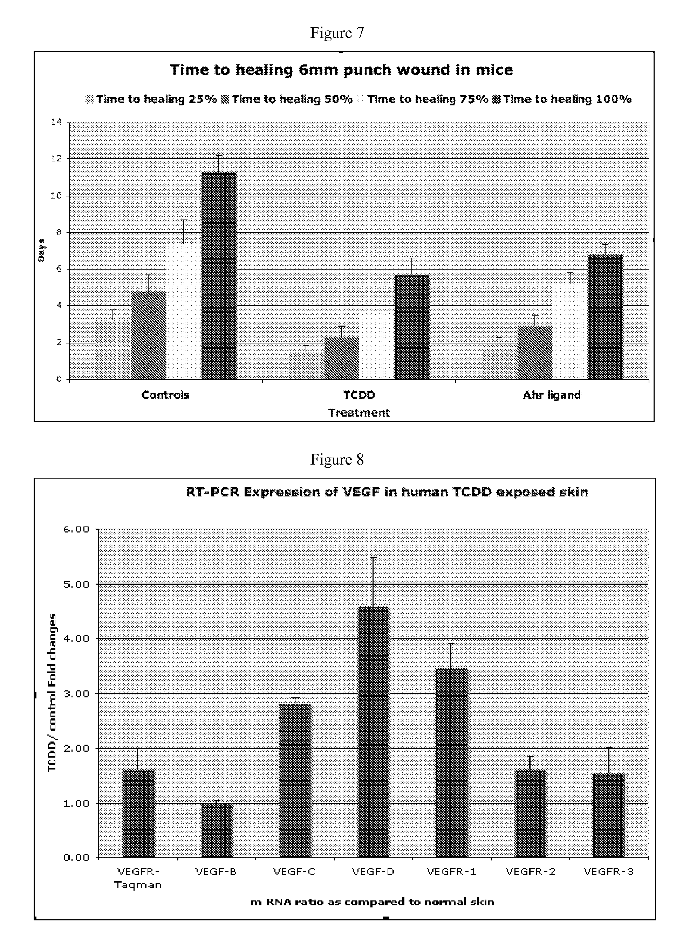

Le temps de cicatrisation de plaies epidermo-dermiques superficielles (dermabrasion) est considérablement diminué par le TCDD. La figure 6a montre le pourcentage de surface cicatrisée en fonction du temps de 4 dermabrasions en cas de présence de TCDD dans la peau (TCDD1-4, 930 à 2900 ppt TCDD mesurée par chromatographie gazeuse/spectrographie de masse) en comparaison avec 4 contrôles (C1 -C4). La figure 6b montre les valeurs moyennes calculées à partir des données de la figure 6a. Ainsi le temps de cicatrisation complète est ramené de 9.3 à 5 jours . La figure 7 montre la diminution du temps de cicatrisation des plaies chez des souris traitées par TCDD ou par Tryptophane irradié par UVB.

L'expression des ARN messagers des facteurs de croissance endothéliale et vasculaire (VEGF) et de leurs récepteurs est augmentée dans la peau exposée au TCDD. La figure 8 montre le rapport (RT PCR) sur fragments de peau humaine contenant 1800 ppt de TCDD, mesurée par chromatographie gazeuse/spectrographie de masse, en comparaison avec les contrôles.

### Exemple 4 : Effet estrogénique

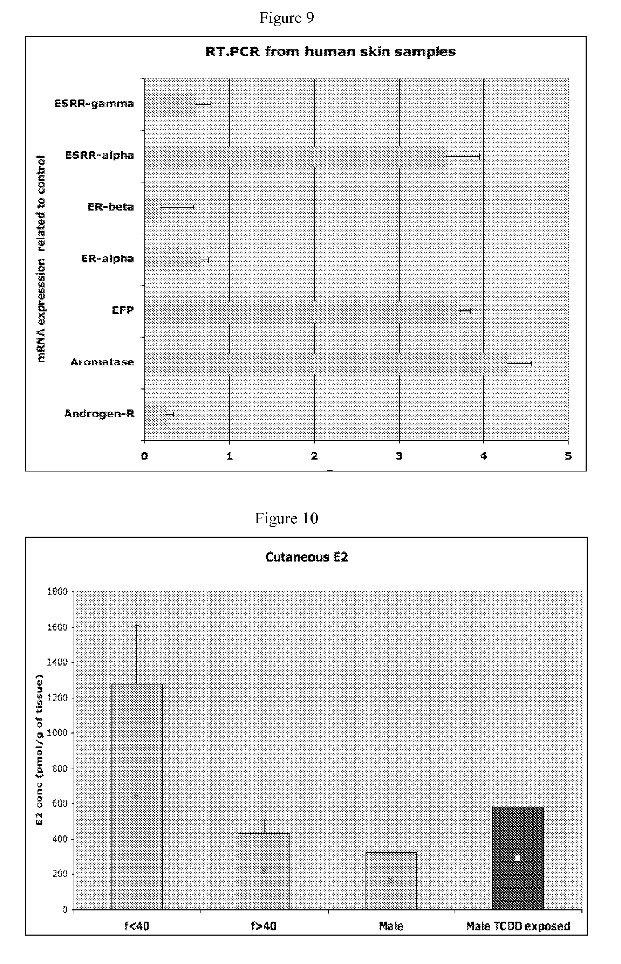

L'expression des ARN messagers des différents récepteurs oestrogéniques ESRR et ER, de la ligase estrogen responsive finger protein (EFP), et de l'enzyme aromatase productrice d'oestrogènes sont augmentée dans la peau exposée au TCDD. La figure 9 montre les rapports RT PCR sur fragments de peau humaine contenant 1800 ppt de TCDD mesurée par chromatographie gazeuse/spectrographie de masse) en comparaison avec les contrôles. La figure 10. montre la concentration de l'oestradiol (E2) dans la peau humaine exposée au TCDD, déterminée par Elisa sur fragments de peau humaine contenant 1800 ppt de TCDD, (mesurée par chromatographie gazeuse/spectrographie de masse) en comparaison avec les contrôles (f<40 femmes de moins de 40 ans ; f>40 femmes de plus de 40 ans, Mâles de 50 ans) .

## Revendications

1. Composition pharmaceutique à usage topique, destinée à induire de façon transitoire la translocation de récepteurs AhR cytosoliques des tissus cutanés vers les noyaux cellulaires des dits tissus, la dite composition comprenant une substance active choisie parmi les ligands agonistes de AhR métabolisables par les enzymes de phase 1 et les précurseurs in situ des dits ligands.

2. Composition selon la revendication 1, **caractérisée en ce que** ladite substance active est choisie parmi les ligands endogènes de AhR.

3. Composition selon la revendication 1, **caractérisée en ce que** ladite substance active est un précurseur choisi parmi les proligands métaboliques de ligands de AhR.

4. Composition selon la revendication 1, **caractérisée en ce que** ladite substance active est un précurseur activable en ligand de AhR sous l'effet d'un agent physique, en particulier sous l'effet d'un rayonnement UV.

5. Composition selon l'une des revendications 1-4, pour le traitement et/ou la prévention de maladies comportant une hypertrophie des glandes sébacées.

6. Composition selon l'une des revendications 1-4, pour le traitement et/ou la prévention d'infections cutanées bactériennes et/ou fongiques.

7. Composition selon l'une des revendications 1-4, pour le traitement et/ou la prévention de la dermatite atopique.

8. Composition selon l'une des revendications 1-4, pour le traitement et/ou la prévention des troubles des fonctions cutanées liés à une deprivation oestrogénique.

9. Composition selon l'une des revendications 1-4, pour le traitement et la cicatrisation des plaies cutanées.

10. Composition selon l'une des revendications 1-4, pour le traitement et/ou la prévention de l'atrophie cutanée.

11. Composition selon l'une quelconque des revendications précédentes **caractérisée en ce que** lesdits ligands ont des durées de demie vie dans l'organisme humain comprises entre 1 et 60 jours, et, plus particulièrement, entre 3 et 30 jours.

12. Composition selon l'une quelconque des revendications précédentes **caractérisée en ce que** ledit ligand est choisi dans le groupe constitué par
• 6-Formylindolo[3,2-b]carbazole et dérivés alkyle ou alkényle du groupe formyle,
• Malassézine,
• Indirubine,
• les Isoflavones avec substituants alkyle, alkényle, alcoxyle ou carboxyle en positions 5, 6 et 3' à 5,'
• le Gallate d'épigallocatéchine; esterifié en l'une ou l'autre des positions 5, 7, 3' à 5', 3" à 5" avec acides gras ou acide rétinoïque,
• le 2-, 3-, 5-, 6- ou 8-Guaïazulénol;le rétinoate ou linoléate de 2-, 3-, 5-, 6- ou 8-guaïazulényle.

13. Composition selon l'une quelconque des revendications 1-11, **caractérisée en ce que** ladite substance active est choisie dans le groupe constitué par leTRP et ses dérivés activables en ligand de AhR sous l'effet d'un rayonnement UV.
